Europäisches Patentamt

European Patent Office

Office européen des brevets

(19)

(11) Numéro de publication : **0 209 436**
**B1**

(12)

# FASCICULE DE BREVET EUROPÉEN

(45) Date de publication du fascicule du brevet :
03.01.90

(21) Numéro de dépôt : 86401428.7

(22) Date de dépôt : 27.06.86

(51) Int. Cl.⁵ : **B 01 J 31/24**, B 01 J 31/16,
C 07 C 2/36, C 07 C 11/08,
C 07 C 11/09

(54) Procédé de préparation d'un catalyseur de dimérisation sélective de l'éthylène ou du propylène en monooléfines du type à base de nickel sur support de silice.

(30) Priorité : 04.07.85 FR 8510235

(43) Date de publication de la demande :
21.01.87 Bulletin 87/04

(45) Mention de la délivrance du brevet :
03.01.90 Bulletin 90/01

(84) Etats contractants désignés :
AT BE CH DE FR GB IT LI LU NL SE

(56) Documents cités :
EP-A- 0 089 895
EP-A- 0 132 840
US-A- 3 697 617
US-A- 4 000 211

(73) Titulaire : **CENTRE NATIONAL DE LA RECHERCHE SCIENTIFIQUE (CNRS)**
**15, Quai Anatole France**
**F-75007 Paris (FR)**

(72) Inventeur : **Lepetit, Christine**
**21, rue Boyer Barret**
**F-75014 Paris (FR)**
Inventeur : **Olivier, Danièle**
**6, rue du Gué**
**F-94400 Villecresnes (FR)**
Inventeur : **Cai, Feng Xian Lab. Réactivité Surface Structure**
**Univ. P.&M.Curie Tour 54 2e étage 4, place Jussieu**
**F-75230 Paris Cédex 05 (FR)**
Inventeur : **Kermarec, Maggy**
**7, rue Jean Jaurès**
**F-92260 Fontenay-aux-Roses (FR)**

(74) Mandataire : **Martin, Jean-Jacques et al**
**Cabinet REGIMBEAU 26, Avenue Kléber**
**F-75116 Paris (FR)**

**Description**

La présente invention concerne la préparation d'un catalyseur de dimérisation sélective d'éthylène ou de propylène en monooléfines, à base de nickel monovalent Ni$^+$ sur support de silice.

Dans la technique antérieure, on connaît déjà un certain nombre de catalyseurs hétérogènes à base de nickel et de silice, qui sont susceptibles de dimériser les oléfines. Comme décrit par Sohn et Osaki dans Journal of Catalysis 59, 303, 1979, ces catalyseurs sont le plus souvent obtenus par co-précipitation en milieu basique d'un silicate et d'un sel de nickel, suivie d'une activation par chauffage entre 100 et 600 °C. Ces catalyseurs ne présentent cependant qu'une faible activité, car le nickel se trouve à l'intérieur de la silice sous une forme difficilement réductible.

La Demanderesse a mis en évidence qu'un catalyseur plus actif peut être obtenu par mise en œuvre d'un mode de préparation permettant précisément d'éviter le passage par un composé de nickel difficilement réductible.

Ce catalyseur, qui fait l'objet de la demande de brevet européenne EP 0089895 au nom de la Demanderesse est caractérisé par la présence de précurseurs de sites d'activité catalytique, constitués par des complexes de nickel monovalent ni$^+$ tricoordiné à trois atomes d'oxygène de la surface du support de silice, ce catalyseur contenant d'environ 0,01 % à environ 1 % en poids sec de Ni$^+$, les complexes de nickel monovalent Ni$^+$ pouvant en particulier comprendre de 1 à 3 ligand(s) de blocage sélectif.

La présente invention concerne une amélioration de la demande antérieure, à savoir du procédé de préparation des catalyseurs de dimérisation sélective de l'éthylène ou du propylène en monooléfines, du type à base de nickel sur support de silice, dont les sites actifs sont des complexes de l'ion Ni$^+$ ; dans ces catalyseurs, pratiquement la totalité du nickel est présent sous la forme de complexes de Ni$^+$ coordiné à l'oxygène de la surface du support de silice et dont la sphère de coordination comprend un ou deux ligands trialkylphosphine.

Selon la présente invention, pratiquement tout le nickel. supporté par la silice, présent pour environ 1 % à environ 6 % en poids sec, est sous forme active Ni$^+$.

Les ligands trialkylphosphine qui occupent, au nombre de 1 ou de 2 selon l'invention, la sphère de coordination de Ni$^+$, sont de préférence la triméthylphosphine (PMe$_3$) ou la triéthylphosphine (PEth$_3$), bien que d'autres ligands sélectifs puissent être envisagés, par exemple la triphénylphosphine ou la tricyclohexylphosphine.

L'introduction dans la sphère de coordination des espèces actives Ni$^+$ d'un nombre défini (1 ou 2) de ligands trialkylphosphine rend les catalyseurs selon l'invention sélectifs pour la dimérisation de l'éthylène ou du propylène en monooléfines. De plus, leur activité est améliorée en intensité et en durabilité par rapport à celle des catalyseurs de l'art antérieur, et leur sélectivité est constante dans le temps.

Les catalyseurs obtenus selon l'invention sont caractérisés par leurs signaux en spectroscopie RPE (résonance paramagnétique électronique) et en spectroscopie UV-visible. Ainsi, en particulier, le catalyseur dont les sites actifs sont des complexes de Ni$^+$ coordiné à deux triéthylphosphines (Ni$^+$(PEth$_3$)$_2$) est caractérisé en RPE par des valeurs d'environ 2,32 (g!) et 2,083 (g$\perp$) et 62 G (A$\perp_1$) et 85 G (A$\perp_2$) des facteurs et des tenseurs hyperfins, et en UV-visible par ses transitions à 465 nm et 330 nm.

Le catalyseur obtenu selon l'invention dont les sites actifs sont des complexes Ni$^+$ coordiné à deux triméthylphosphines (Ni$^+$(PMe$_3$)$_2$) est caractérisé en RPE par des valeurs d'environ 2,26 (g!) et 2,077 (g$\perp$) et 60 G (A$\perp_1$) 75 G (A$\perp_2$) des facteurs et tenseurs hyperfins, et en UV-visible par ses transitions à 450 nm et 327 nm.

Enfin, le catalyseur obtenu selon l'invention dont les sites actifs sont des complexes de Ni$^+$ coordiné à une triéthylphosphine (Ni$^+$(PEth$_3$)) est caractérisé en RPE par des valeurs d'environ 2,013 (g!) et 2,125 (g$\perp$) et 70 G (A$\perp$) des facteurs et tenseurs hyperfins et en UV-visible par sa transition à 400 nm.

Lorsque les sites actifs sont des complexes de Ni$^+$ coordiné à une triméthylphosphine, (Ni$^+$(PMe$_3$)) est caractérisé en RPE par des valeurs d'environ 2,015 (g!) et 2,120 (g$\perp$) et 55 G (A$\perp$) des facteurs et tenseurs hyperfins et en UV-visible par sa transition à 400 nm.

Le catalyseur obtenu selon l'invention dont les sites actifs sont des complexes de Ni$^+$ coordiné à une triméthylphosphine (Ni$^+$(PMe$_3$)) est caractérisé en RPE par des valeurs d'environ 2,015 (g!) et 2,120 (g$\perp$) et 55 G (A$\perp$) du facteur et tenseurs hyperfins, et en UV-visible par sa transition à 400 nm.

La présente invention concerne un procédé de fabrication des catalyseurs de dimérisation caractérisé en ce qu'il comporte les différentes étapes successives suivantes :

a) on traite la silice par une solution aqueuse d'un composé générateur de cations monovalents, à un pH supérieur à environ 10, de manière à fixer au moins une partie desdits cations monovalents sur la silice ;

b) on traite la silice ainsi obtenue par une solution aqueuse d'un sel de nickel divalent et d'un agent complexant du sel de nickel à un pH au moins égal à environ 8, de manière à échanger lesdits cations monovalents portés par la silice avec des cations de nickel divalents ;

c) on porte la silice ainsi modifiée à une température comprise entre 300 °C et 900 °C,

d) on traite la silice ainsi obtenue par une trialkylphosphine ou l'un de ses précurseurs en excès jusqu'à l'obtention de complexes Ni$^+$ coordiné avec quatre ligands trialkylphosphine, et

e) on traite sous vide la silice résultante à une température appropriée et durant une durée appropriée pour obtenir des sites Ni$^+$ actifs dont la sphère de coordination comporte au plus deux ligands trialkylphosphine.

D'autres caractéristiques et avantages de la présente invention apparaîtront à la lecture de la description détaillée, des exemples ci-après, et sur les dessins annexés.

Le catalyseur de dimérisation est obtenu par la mise en œuvre d'un procédé qui se décompose en cinq étapes successives, présentées ci-après de façon détaillée.

a) Fixation de cations monovalents sur une silice

On traite la silice par une solution aqueuse d'un composé fournissant, en solution aqueuse, des cations monovalents, notamment des cations de métaux alcalins, d'ammonium ou d'ammonium quaternaire, par exemple $NH_4^+$, $Na^+$, $Li^+$, $K^+$ ou $NR_4^+$ (R = reste monovalent hydrocarboné), à un pH supérieur à environ 10, de préférence entre environ 11 et environ 11,8.

Des exemples de composés fournissant des cations monovalents en solution, dans le domaine de pH précité, sont la soude, la potasse, l'ammoniaque et les hydroxides d'ammonium quaternaire, avec ou sans sels additionnels, par exemple le chlorure d'ammonium, le nitrate de sodium, le chlorure de lithium, l'acétate d'ammonium ou un chlorure ou sulfate d'ammonium quaternaire.

Cette étape de fixation est avantageusement effectuée à une température comprise entre environ 0 et environ 80 °C, de préférence entre environ 20 et environ 60 °C, en opérant, par exemple, dans un récipient sous agitation ou par circulation de la solution aqueuse au travers d'un lit de silice. La durée du traitement peut s'étendre, par exemple, de 3 heures à 3 jours.

Au cours de cette étape de fixation, le pH doit demeurer supérieur à environ 10 et de préférence être compris entre environ 11 et environ 11,8, ce qui peut nécessiter dans certains cas l'addition complémentaire d'une base telle que la soude, la potasse ou l'ammoniaque si l'on constate une baisse excessive du pH. Pour éviter cet inconvénient, une méthode préférée consiste à utiliser la solution aqueuse en quantité et concentration suffisantes pour que le pH ne baisse pas de plus d'environ 0,3 unité.

Pour ce premier traitement, la silice peut être mise en œuvre sous une forme quelconque, et notamment sous forme de poudre, de billes ou d'extrudés.

b) Echange compétitif des cations monovalents avec le cation Ni$^{2+}$

La silice traitée comme indiquée ci-dessus et qui renferme des cations monovalents est traitée par une solution aqueuse d'un sel de nickel bivalent et d'un agent complexant du sel de nickel, à un pH d'au moins environ 8, et de préférence compris entre environ 9 et environ 11,8. L'agent complexant basique est avantageusement l'ammoniac ou une amine, par exemple la pyridine, la monoéthyl amine, la diéthyl amine ou la triméthyl amine. La solution contient, par exemple d'environ 0,001 à environ 1 mole de sel de nickel par litre, la concentration et la quantité de solution étant choisies en fonction du nombre d'ions monovalents qu'on désire échanger de manière compétitive. Pour donner un ordre de grandeur, un litre de cette solution permet de traiter entre environ 1 et environ 100 g de silice. La durée du traitement est comprise par exemple entre environ 3 heures et environ 3 jours, selon le taux d'échange compétitif désiré, et peut se faire par exemple dans un récipient sous agitation ou par circulation de la solution au travers d'un lit de silice. La température de l'échange compétitif est avantageusement comprise entre environ 0 et environ 80 °C. La silice modifiée ainsi obtenue est séparée de la phase aqueuse et lavée à l'eau. Elle contient par exemple, entre environ 0,01 et environ 10 % en poids sec de nickel.

Il est préférable de limiter le taux d'échange compétitif en nickel à environ 7 % afin d'éviter la formation de nickel métallique au cours de la réduction ultérieure. On fixe de préférence ainsi 1 à 6 % en poids de nickel sur la silice.

c) Prétraitement thermique

La silice obtenue au cours de l'étape précédente est ensuite portée à une température comprise entre environ 300 et environ 900 °C, de préférence entre environ 500 et environ 700 °C. On peut opérer sous vide, dans un gaz inerte, tel que l'azote ou l'argon, ou de préférence dans un gaz oxydant, tel que l'oxygène pur ou un mélange d'oxygène avec un gaz inerte. Selon l'invention, on préfère traiter sous vide, à environ 700°. La montée en température est de préférence progressive, sa vitesse étant, par exemple, comprise entre environ 10 et environ 500 °C par heure. La durée du prétraitement thermique est, par exemple d'environ 1 à environ 12 heures. Ce traitement permet d'éliminer l'eau contenue dans la silice. Si le chauffage de la silice se fait par l'intermédiaire de la phase gazeuse le débit de gaz est réglé en fonction de la température à atteindre. A la fin du prétraitement thermique, la silice est refroidie à l'abri de l'humidité.

d) Transformation des ions Ni$^{2+}$ en Ni$^+$

La silice obtenue au terme du prétraitement thermique est ensuite soumise à un traitement par une trialkylphosphine ou un précurseur de trialkylphosphine, ce traitement pouvant être mené à température ambiante, en phase gazeuse ou en phase liquide. La quantité de phosphine utilisée est en excès par rapport au nickel supporté. Le rapport trialkylphosphine/Ni est avantageusement compris entre environ 3 et environ 6. Quand la transformation est réalisée en phase gazeuse, la pression de phosphine est de préférence comprise entre environ 6 650 et 13 500 Pa. La réduction peut être suivie et contrôlée par spectroscopie RPE et UV-visible à partir de l'augmentation de l'intensité des signaux des espèces $Ni^+$ $(Palk_3)_4$ formées, et dure de 1 à 2 heures en phase gazeuse.

En phase liquide, cette réduction est menée dans un solvant des phosphines, par exemple du toluène ou de l'heptane. La réduction est immédiate en milieu solvant.

Comme précurseur de trialkylphosphine, on peut utiliser par exemple, du bis(diméthylphosphine) méthane ou du bis(diéthylphosphine) méthane.

Les caractéristiques en RPE et en UV-visible de l'espèce $Ni^+(Palk_3)_4$ sont les suivantes :

| alk | RPE | | | | UV-vis nm |
|-----|-----|-----|-----|-----|-----------|
| | g | | A (gauss) | | |
| | ‖ | ⊥ | ‖ | ⊥ | |
| Eth | 2,27 | 2,065 | 40· | 50 | 1 400 |
| Me | 2,21 | 2,052 | 40 | 50 | 1.175 |

On peut en outre montrer que la réduction des ions $Ni^{2+}$ en $Ni^+$ est complète par la disparition des bandes caractéristiques de $Ni^{2+}$ sur les spectres UV-visible annexés. Sur ces spectres, les bandes caractéristiques de $Ni^{2+}$ apparaissent à environ 700 nanomètres.

e) Activation du catalyseur conduisant à l'obtention des espèces actives $Ni^+$ $(Palk_3)_2$ et $Ni^+$ $(Palk_3)$

Ces espèces sont obtenues par traitement sous vide du catalyseur précédent comportant les espèces $Ni^+$ $(Palk_3)_4$ à une température qui dépend de la nature de la trialkylphosphine, de préférence pendant 30 à 60 minutes.

Les températures auxquelles on opère l'évacuation du catalyseur en fonction de la trialkylphosphine et du nombre de ligands trialkylphosphine que l'on conserve dans la sphère de coordination du $Ni^+$, sont les suivantes :

| | |
|---|---|
| $Ni^+(PEth_3)_2$ | 50 à 150 °C |
| $Ni^+(PMe_3)_2$ | 300 à 400 °C |
| $Ni^+(PEth_3)$ | 150 à 250 °C |
| $Ni^+(P(C_6H_{11})_3)$ | température ambiante |

La dimérisation sélective s'effectue par mise en contact de l'éthylène ou du propylène avec le catalyseur obtenu. L'oléfine peut être utilisée en phase gazeuse ou en phase liquide, à l'état pur ou en solution dans un solvant. Les meilleurs solvants de la réaction sont les hydrocarbures paraffiniques ou aromatiques, ou leurs dérivés chlorés, tels le pentane, l'heptane, le benzène, le toluène, le chlorure de méthylène, le dichloréthane, le chlorobenzène.

La température de dimérisation est avantageusement comprise entre environ 0° et environ 100 °C, de préférence entre environ 30 et 40 °C et la pression est choisie pour maintenir une phase gazeuse ou liquide. La pression peut varier entre $10^5$ et $50.10^5$ Pa, de préférence entre environ $20.10^5$ Pa et environ $30.10^5$ Pa. La dimérisation peut être conduite en système fermé (on introduit en une seule fois le catalyseur et l'oléfine dans le réacteur), ou en système ouvert (on fait passer l'oléfine au travers d'un lit du catalyseur), les meilleurs résultats étant obtenus en système ouvert. Le temps de contact entre le réactif et le catalyseur est usuellement compris entre environ 1 minute et environ 24 heures.

De préférence, la dimérisation de l'éthylène est réalisée sur des catalyseurs à sites actifs comportant un ou deux ligands trialkylphosphine, et on obtient alors sélectivement du butène-1, et la dimérisation du propylène sur les catalyseurs dont les sites actifs comportent un ligand trialkylphosphine, pour obtenir sélectivement des monooléfines.

De préférence, dans les catalyseurs employés, les trialkylphosphines sont des triéthylphosphines.

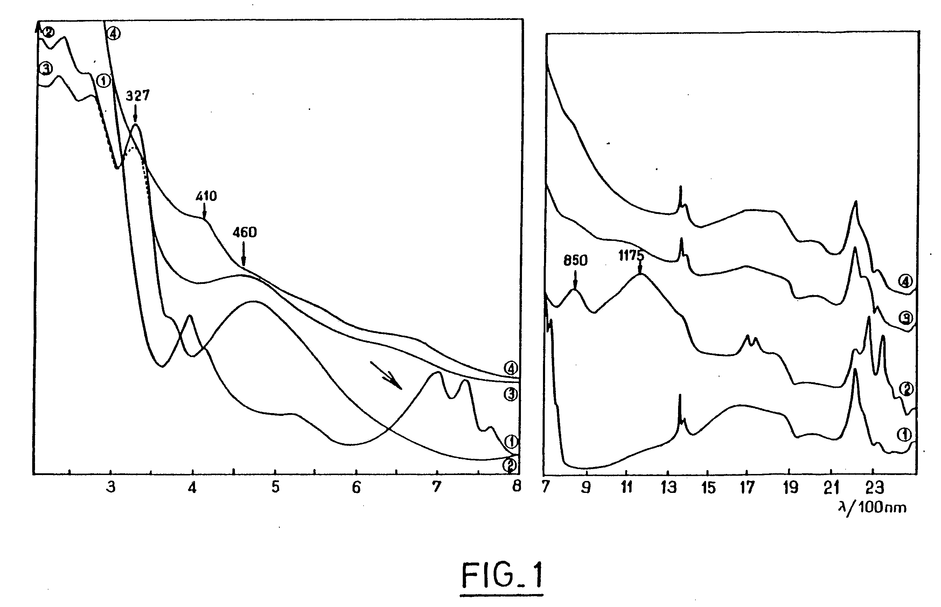

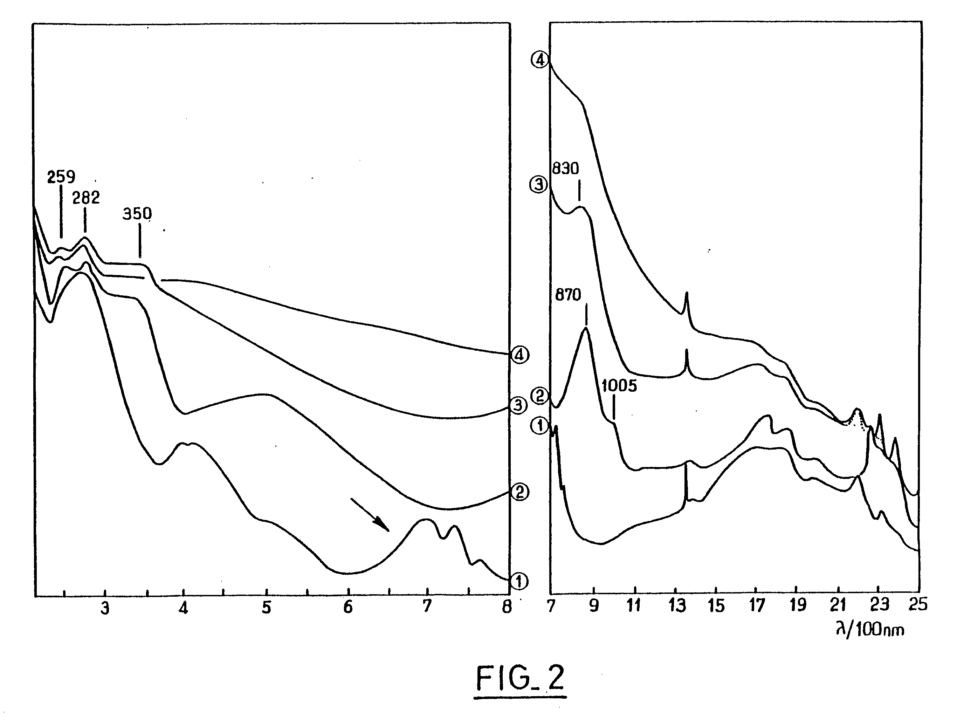

Les figures 1 et 2 représentent les spectres des catalyseurs obtenus selon l'invention dans le cas d'une réduction par $PMe_3$ (figure 1) et par $PEth_3$ (figure 2).

Sur la figure 1 : (1) correspond au catalyseur non réduit issu de l'étape c), (2) au catalyseur traité issu de l'étape d), (3) au catalyseur évacué à 200 °C, (4) le catalyseur évacué à 300 °C pendant 15 mn.

Sur la figure 2 : (1) correspond au catalyseur non réduit issu de c), (2) au catalyseur traité issu de d), (3) au catalyseur traité sous vide à 150° pendant 30 minutes, (4) au catalyseur traité sous vide à 250 °C pendant 35 mn.

Sur ces deux figures, les bandes autour de 700 nm, signalées par une flèche, disparaissent en cours des étapes d) et e) ce qui montre la réduction totale de $Ni^{2+}$ en $Ni^+$.

Les figures 3, 4 et 5 présentent des résultats de dimérisation de l'éthylène et du propylène et seront décrits plus précisément dans les exemples.

### Exemple 1 : (étapes a et b)

30 g de silice sont traités à température ambiante par 1 litre de solution aqueuse d'ammoniac à pH 11,6, sous faible agitation pendant 24 heures. On vérifie que la baisse du pH est restée inférieure à 0,3 unité. La silice utilisée est : soit une silice microporeuse (diamètre moyen des pores 5 nm) soit une sphérosil (diamètre moyen des pores 8 nm), les deux silices étant commercialisées par la Société Rhône-Poulenc. Après filtration, les échantillons ainsi traités sont ensuite mis en contact à température ambiante avec 1 litre d'une solution contenant des ions $Ni(NH_3)_6^{2+}$, obtenue par dissolution de 0,05 mole de nitrate de nickel dans une solution aqueuse contenant 0,3 mole de nitrate d'ammonium et 1,5 mole d'ammoniac et dont le pH est voisin de 9,8. L'agitation est poursuivie pendant 60 heures à température ambiante. La silice est filtrée puis lavée à l'eau déionisée, séchée à 50 °C sous vide. L'analyse élémentaire montre que sa teneur en nickel est de 1,7 % en poids sec.

### Exemple 2 : (étapes a et b)

17 g de silice sont traités par une solution aqueuse d'ammoniac dans les conditions décrites dans la première étape de l'exemple 1. Après filtration, la silice est mise dans 1 litre d'une solution contenant des ions $Ni(HH_3)_6^{2+}$ préparée à partir de la dissolution de 0,1 mole de nitrate de nickel et de 0,6 mole de nitrate d'ammonium dans une solution aqueuse contenant 4 moles d'ammoniac. La solution a un pH voisin de 10,4. Après 60 heures d'agitation à température ambiante la silice est lavée, filtrée et séchée comme dans l'exemple 1. Elle titre 4,2 % en nickel pour le support de silice microporeuse RP sous forme d'extrudés et 4,9 % en nickel dans le cas du support spherosil Pechiney-St-Gobain.

### Exemple 3 : (étape c)

Les échantillons 1 ou 2 tels qu'obtenus aux exemples 1 ou 2 sont placés dans un tube sous vide et chauffés progressivement jusqu'à 700 °C. Le tube est ensuite refroidi à température ambiante.

### Exemple 4 : (étapes d et e)

400 mg d'échantillons 1 ou 2 tels qu'obtenus à l'exemple 3 sont transvasés sous vide dans un volume de 50 cm³, puis traités 2 heures à température ambiante sous une pression de 7 980 Pa environ de triéthylphosphine. Les échantillons sont ensuite évaporés sous vide pendant 15 minutes à température ambiante, puis pendant 1 heure à 100 °C. Ils sont ensuite refroidis sous vide jusqu'à la température ambiante. On obtient ainsi les catalyseurs au Ni $(PEth_3)_2$. Ces catalyseurs sont de préférence conservés sous vide.

### Exemple 5 : (étapes d et e)

Pour obtenir les catalyseurs au Ni $(PEth_3)$, on traite 400 mg des échantillons des catalyseurs 1 ou 2 de la même façon qu'à l'exemple 4 jusqu'à l'évaporation sous vide qui est menée pendant 15 minutes à température ambiante, puis 1 heure à 200 °C. Ces catalyseurs sont également de préférence conservés sous vide.

### Exemple 6 : (étapes d et e)

Pour réaliser des catalyseurs au $Ni(PMe_3)_2$, on procède de la même façon qu'aux exemples 4 et 5, en partant de 400 mg d'échantillons 1 ou 2 obtenus selon l'exemple 3, en traitant d'abord sous pression d'environ 7 980 Pa de triméthylphosphine, puis en évacuant sous vide 15 minutes à température ambiante, puis 1 heure à 350 °C. Ces catalyseurs sont également conservés sous vide de préférence.

### Exemple 7 : (étapes d et e)

Pour réaliser des catalyseurs au Ni $(P(C_6H_{11})_3)$, on procède de la même façon qu'aux exemples

5

précédents, l'évacuation s'effectuant sous vide pendant une heure environ à température ambiante.

Exemple 8

Dimérisation de l'éthylène en 1-butène

Cet exemple a été conduit en régime dynamique (ouvert) sur environ 150 mg de catalyseur. Le tableau annexé montre les résultats obtenus pour les catalyseurs produits selon l'invention, en comparaison avec les catalyseurs connus au $Ni^+$, dans différentes conditions expérimentales.

Ces résultats rassemblés sur le tableau 1 ci-après montrent la stabilité beaucoup plus importante des catalyseurs obtenus selon l'invention, ainsi qu'une stabilité en butène-1 avantageuse.

Exemple 9

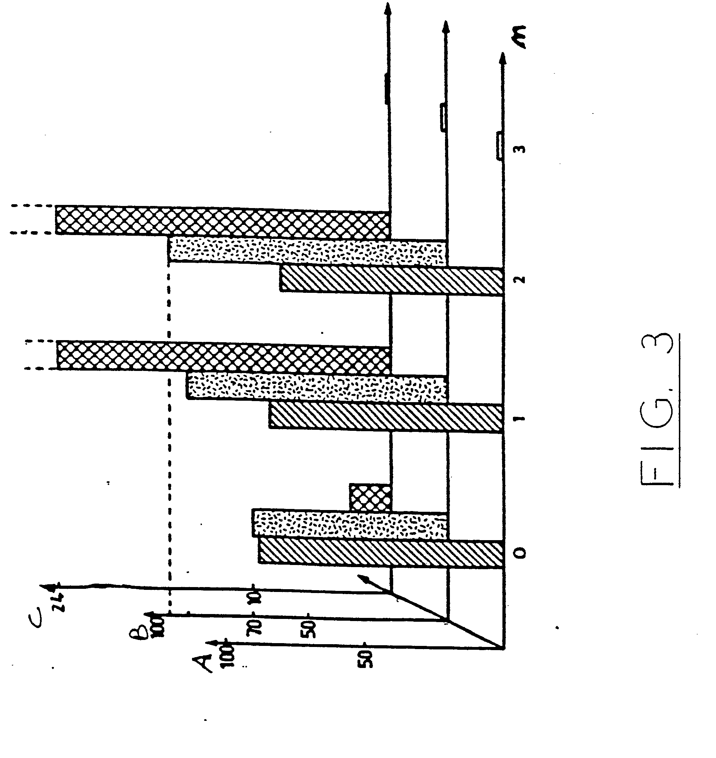

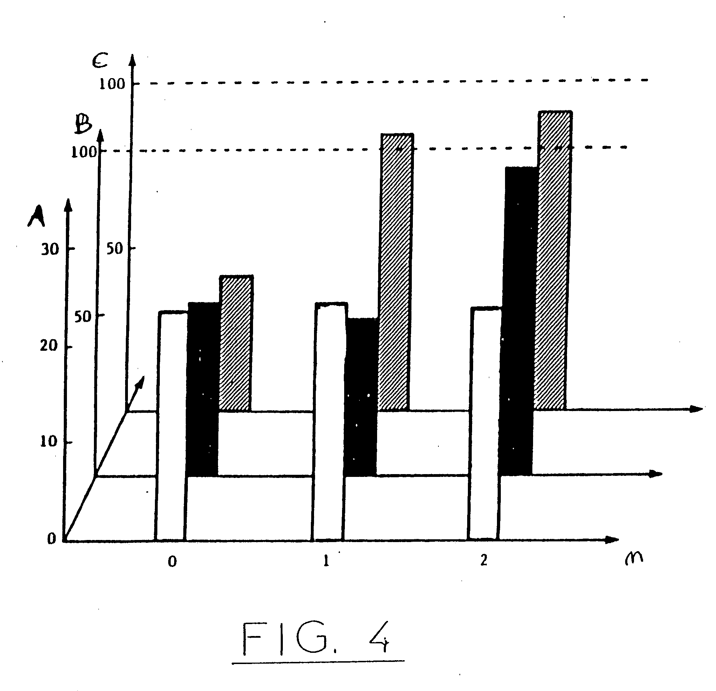

D'autres exemples de dimérisation de l'éthylène en 1-butène ont été réalisés, dont les résultats apparaissent sur les diagrammes des figures 3 et 4.

Pour une pression de $20.10^5$ Pa, à 40 °C, et à un taux de conversion de 5 % en régime dynamique, le diagramme de la figure 3 représente la sélectivité (%) (A), le pourcentage molaire de 1-butène obtenu (B) et la stabilité en heures (C), ceci pour des catalyseurs $Ni^+(PEth_3)$, c'est-à-dire des catalyseurs connus (n = 0), en comparaison avec les catalyseurs selon l'invention (n = 1 et n = 2), ainsi que des catalyseurs où la sphère de coordination comporte 3 ligands triéthylphosphine (u = 3).

Pour une pression de $20.10^5$ Pa, à 25 °C, dans un réacteur de 100 ml en régime statique, pour des catalyseurs $Ni^+(PEth_3)_n$ (n = 0, n = 1 et n = 3), le diagramme de la figure 4 représente le taux de conversion (A), le pourcentage molaire de 1-butène obtenu (B) et la sélectivité en dimère (C).

6

EP 0 209 436 B1

Tableau I

| Catalyseur | P en Pa | T° C | Débit $lh^{-1}$ | Conversion mole $C_2H_4$ % | nombre de rotation mole $C_2H_4 Ni^{-1}h^{-1}$ | Stabilité h | Sélectivité en dimères % | Distribution des $C_4$ mole % | |
|---|---|---|---|---|---|---|---|---|---|
| | | | | | | | | 1 butène | 2 butène |
| $Ni^+$ | $20.10^5$ | 40 | 12 | 10 | 317 | 3 | 88 | 71 | 29 |
| $Ni^+$ | $20.10^5$ | 40 | 3 | 37 | 277 | 3 | 82 | 51 | 49 |
| $Ni^+(PEth_3)_2$ | $20.10^5$ | 40 | 3 | 5,5 | 63,7 | > 24 | 80 | 94 | 6 |
| $Ni^+(PEth_3)$ | $20.10^5$ | 40 | 3 | 5,3 | 32,3 | > 24 | 84 | 93 | 7 |
| $Ni^+(PEth_3)_2$ | $20.10^5$ | 40 | 1,3 | 9,1 | 45 | > 24 | 83 | 95 | 5 |

D'après ces résultats, il apparaît que les catalyseurs obtenus selon l'invention (n = 1 et n = 2) sont stables plus de 24 h, contrairement aux catalyseurs pour lesquels n égal 0 ou 3.

De plus, même à des taux de conversion élevé, la sélectivité ou dimère reste bonne pour les catalyseurs obtenus selon l'invention.

Il ressort clairement de ces résultats que la présence de deux ligands de blocage sélectifs modifie de façon considérable la sélectivité du catalyseur obtenu selon l'invention : la sélectivité pour l'obtention de 1-butène à partir d'éthylène est supérieure à 95 % pour une conversion de 25 %.

Exemple 10

Dimérisation du propylène

On a également réalisé avec les catalyseurs obtenus selon l'invention la dimérisation du propylène, pour laquelle les catalyseurs se sont également révélés beaucoup plus stables que les catalyseurs connus. De plus, ils sont réutilisables.

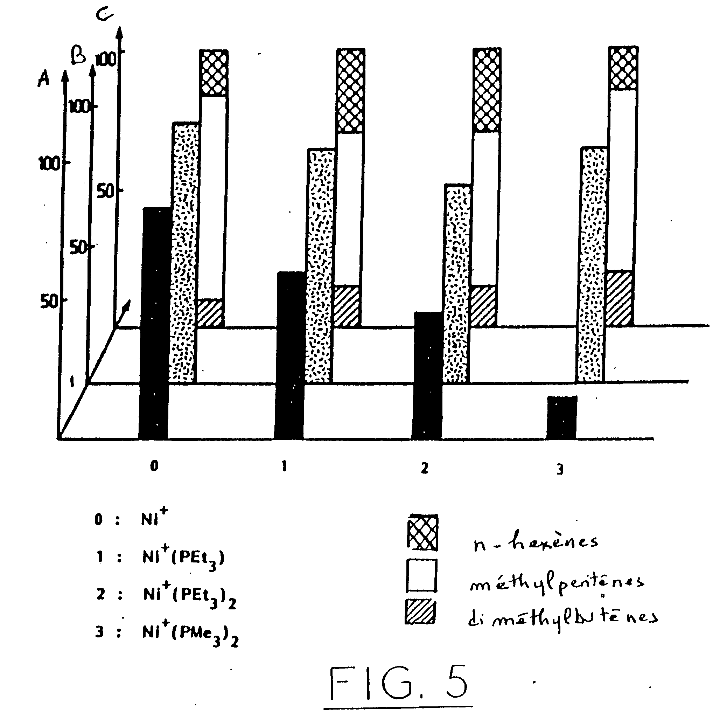

Ainsi, le diagramme de la figure 5 donne les résultats de conversion (A) de sélectivité en dimères en $C_6$ (B) et de distribution de dimères obtenus (C) pour chacun des catalyseurs indiqués.

Le dimérisation du propylène a été réalisée en régime statique à une pression atmosphérique et une température de 25 °C.

Ces résultats montrent que l'on peut obtenir des taux de conversion de l'ordre de 50 % dans certains cas et montrent l'effet du nombre et de la nature des ligands sur le taux de conversion.

Exemple 11

Le tableau suivant établit la comparaison entre les produits réactionnels de la dimérisation du propylène lorsque l'on utilise un catalyseur connu, un catalyseur contenant un ligand triéthylphosphine ou cyclohexylphosphine.

Les conditions expérimentales sont les suivantes :

— température de réaction : 40 °C,
— pression initiale : $5.10^5$ Pa,
— catalyseur à 2,1 % en poids de nickel,
— conversion 25 %,
— régime statique.

Tableau II

| MOLE % | $Ni^+$ | $Ni^+(PEth_3)$ | $Ni^+(P(C_6H_{11})_3)$ |
|---|---|---|---|
| 1-hexène + 2 méthyl-1-propène | 4,7 | 9 | 4,8 |
| transhexène + 2 méthyl-2-pentène | 39,6 | 29,8 | 35,2 |
| Cis 2-hexène | 4,5 | 0,9 | 4,6 |
| 3-hexène | 4,2 | 0,2 | 3,7 |
| 4 méthyl-1-pentène | 3,8 | 1,5 | 5,1 |
| 2,3-diméthyl-1-butène + 4 méthyl-2 pentène cis | 9 | 25,9 | 13,4 |
| 4 méthyl-2-pentène trans | 27 | 22 | 27 |
| 2,3 diméthyl -2-butène | 6,7 | 9,4 | 6,2 |

Exemple 12

Dimérisation du propylène

On a également comparé la sélectivité en dimère $C_6$, en régime statique, dans les mêmes conditions de température et de pression, à un taux de conversion de 50 % et pour des catalyseurs usuels $Ni^+$ et des catalyseurs $Ni^+(P(C_6H_{11})_3)$. La sélectivité en dimère $C_6$ passe de 30 % en l'absence de phosphine à 53 % en présence de tricyclohexinephosphine.

**Revendications**

1. Procédé de fabrication d'un catalyseur de dimérisation sélective de l'éthylène ou du propylène en monooléfines, du type à base de nickel sur support de silice, dont les sites actifs sont des complexes de l'ion Ni⁺, caractérisé en ce que l'on effectue les différentes étapes successives suivantes :

a) on traite la silice par une solution aqueuse d'un composé générateur de cations monovalents, à un pH supérieur à environ 10, de manière à fixer au moins une partie desdits cations monovalents sur la silice ;

b) on traite la silice ainsi obtenue par une solution aqueuse d'un sel de nickel divalent et d'un agent complexant du sel de nickel à un pH au moins égal à environ 8, pour échanger de manière compétitive lesdits cations monovalents portés par la silice avec des cations de nickel divalents ;

c) on porte la silice ainsi modifiée à une température comprise entre environ 300 et environ 900 °C ;

d) on traite la silice ainsi obtenue par une trialkylphosphine ou l'un de ses précurseurs en excès jusqu'à l'obtention de complexes de Ni⁺ coordiné avec quatre ligands trialkylphosphine, et

e) on traite sous vide la silice résultante à une température appropriée et durant une durée appropriée pour obtenir des sites Ni⁺ actifs dont la sphère de coordination comporte au plus deux ligands trialkylphosphine.

2. Procédé selon la revendication 1, caractérisé en ce que l'étape c) est menée à environ 700 °C.

3. Procédé selon l'une des revendications 1 ou 2 caractérisé en ce que l'étape c) est menée sous vide.

4. Procédé selon l'une des revendications 1 à 3 caractérisé en ce qu'à l'étape d), on traite la silice par une triméthylphosphine, une triéthylphosphine, du bis(diméthylphosphine) méthane ou du bis(diéthyl-phosphine) méthane.

5. Procédé selon l'une des revendications 1 à 4, caractérisé en ce qu'à l'étape d), l'excès de réactif utilisé correspond à un rapport trialkylphosphine/Ni allant d'environ 3 à environ 6.

6. Procédé selon l'une des revendications 1 à 5, caractérisé en ce qu'à l'étape d) on traite la silice en phase liquide dans un solvant des trialkylphosphines, ou en phase gazeuse, et à température ambiante.

7. Procédé selon l'une des revendications 1 à 6, caractérisé en ce qu'à l'étape d) le solvant est le toluène ou l'heptane.

8. Procédé selon l'une des revendications 1 à 6, caractérisé en ce qu'à l'étape d) la pression de phosphine est de l'ordre de 6 650 à 13 500 Pa.

9. Procédé selon l'une des revendications 1 à 6, caractérisé en ce que l'étape e) est menée entre environ 50° et 250 °C, pendant une durée allant d'environ 30 min à environ 1 heure pour obtenir des sites Ni⁺ actifs dont la sphère de coordination comporte un ou deux ligands triéthylphosphine.

10. Procédé selon l'une des revendications 1 à 6, caractérisé en ce que l'étape e) est menée entre environ 300° et 400 °C pendant une durée allant d'environ 30 min à environ 1 heure pour obtenir des sites Ni⁺ actifs dont la sphère de coordination comporte deux ligands triméthylphosphine.

11. Procédé selon l'une des revendications 1 à 6, caractérisé en ce que l'étape e) est menée à température ambiante pendant une durée d'environ une heure pour obtenir des sites Ni⁺ actifs dont la sphère de coordination comporte un ligand tricyclohexylphosphine.

**Claims**

1. Process for the manufacture of a catalyst for the selective dimerisation of ethylene or propylene into monoolefines of the type based on nickel on a silica support, whose active sites are complexes of the Ni⁺ ion comprising performing the following different successive steps :

a) treating silica with an aqueous solution of a compound generating monovalent cations, at a pH higher than about 10, so as to fix at least a part of said monovalent cations to the silica ;

b) treating the silica thus obtained with an aqueous solution of a divalent nickel salt and a complexing agent for the nickel salt at a pH at least equal to about 8, to exchange competitively said monovalent cations borne by the silica with divalent nickel cations ;

c) bringing the so-modified silica to a temperature comprised between about 300 and about 900 °C ;

d) treating the so-obtained silica with a trialkylphosphine or one of its precursors in excess until the production of complexes of the Ni⁺ co-ordinated with four trialkylphosphine ligands, and

e) treating the resulting silica under vacuum at a suitable temperature and for an appropriate time to obtain active Ni sites whose co-ordination sphere comprises at the most two trialkylphosphine ligands.

2. Process according to claim 1, wherein step c) is performed at about 700 °C.

3. Process according to claims 1 or 2, wherein step c) is performed under vacuum.

4. Process according to claims 1 to 3, wherein at step d), the silica is treated with a trimethylphosphine a triethylphosphine, bis(dimethylphosphine) methane or bis(diethylphosphine) methane.

5. Process according to claims 1 to 4, wherein at step d) the excess reactant used corresponds to a trialkylphosphine/Ni ratio ranging from about 3 to about 6.

6. Process according to claims 1 to 5, wherein at step d) the silica is treated in liquid phase in a solvent for trialkylphosphines, or in gaseous phase, and at ambient temperature.

7. Process according to claims 1 to 6, wherein at step d) the solvent is toluene or heptane.

8. Process according to claims 1 to 6, wherein at step d) the pressure of phosphine is of the order of 6 650 to 13 500 Pa.

9. Process according to claims 1 to 6, wherein step e) is performed between about 50° and 250 °C, for a time ranging from about 30 min to about 1 hour to obtain active Ni$^+$ sites whose co-ordination sphere comprises one or two triethylphosphine ligands.

10. Process according to claims 1 to 6, wherein step e) is performed between about 300° and 400 °C for a time ranging from about 30 min to about 1 hour to obtain active Ni$^+$ sites whose co-ordination sphere comprises two trimethylphosphine ligands.

11. Process according to claims 1 to 6, wherein step e) is performed at a surrounding temperature for a period of about 1 hour to obtain the active Ni$^+$ sites whose co-ordination sphere comprises one tricyclohexylphosphine ligand.


**Patentansprüche**

1. Verfahren zur Herstellung eines Katalysators vom Typ auf der Basis von Nickel auf einem Kieselsäureträger für die selektive Dimerisierung von Ethylen oder Propylen zu Monoolefinen, dessen aktive Stellen Komplexe des Ions Ni$^+$ sind, dadurch gekennzeichnet, daß man die folgenden verschiedenen Schritte nacheinander durchführt :

a) man behandelt die Kieselsäure mit einer wässrigen Lösung einer Verbindung, die einwertige Kationen erzeugt, bei einem pH größer als etwa 10, so daß wenigstens ein Teil besagter einwertiger Kationen auf der Kieselsäure fixiert werden ;

b) man behandelt die so erhaltene Kieselsäure mit einer wässrigen Lösung eines Nickel(II)-Salzes und eines Komplexbildners des Nickelsalzes bei einem pH wenigstens gleich ungefähr 8, um in kompetitiver Weise besagte einwertige Kationen, die von der Kieselsäure getragen werden, gegen die Kationen des zweiwertigen Nickels auszutauschen ;

c) man bringt die so modifizierte Kieselsäure auf eine Temperatur zwischen ungefähr 300 und ungefähr 900 °C ;

d) man behandelt die so erhaltene Kieselsäure mit einem Trialkylphosphin oder einem von dessen Vorläufern im Überschuß, bis man Komplexe von mit vier Trialkylphosphin-Liganden koordiniertem Ni$^+$ erhält ; und

e) man behandelt die resultierende Kieselsäure im Vakuum bei einer geeigneten Temperatur und über einen geeigneten Zeitraum, um aktive Ni$^+$-Stellen zu erhalten, deren Koordinationsphäre höchstens zwei Trialkylphosphin-Liganden aufweist.

2. Verfahren nach Anspruch 1, dadurch gekennzeichnet, daß der Schritt c) bei etwa 700 °C durchgeführt wird.

3. Verfahren nach einem der Ansprüche 1 oder 2, dadurch gekennzeichnet, daß der Schritt c) im Vakuum durchgeführt wird.

4. Verfahren nach einem der Ansprüche 1 bis 3, dadurch gekennzeichnet, daß man beim Schritt d) die Kieselsäure mit einem Trimethylphosphin, einem Triethylphosphin, Bis(dimethylphosphin)methan oder Bis(diethylphosphin)methan behandelt.

5. Verfahren nach einem der Ansprüche 1 bis 4, dadurch gekennzeichnet, daß bei Schritt d) der Überschuß des verwendeten Reagens einem Verhältnis Trialkylphosphin/Ni entspricht, das von etwa 3 bis etwa 6 reicht.

6. Verfahren nach einem der Ansprüche 1 bis 5, dadurch gekennzeichnet, daß man bei Schritt d) die Kieselsäure in flüssiger Phase in einem Lösungsmittel der Trialkylphosphine oder in gasförmiger Phase, und bei Raumtemperatur behandelt.

7. Verfahren nach einem der Ansprüche 1 bis 6, dadurch gekennzeichnet, daß bei Schritt d) das Lösungsmittel Toluol oder Heptan ist.

8. Verfahren nach einem der Ansprüche 1 bis 6, dadurch gekennzeichnet, daß bei Schritt d) der Phosphindruck in der Größenordnung von 6 650 bis 13 500 Pa liegt.

9. Verfahren nach einem der Ansprüche 1 bis 6, dadurch gekennzeichnet, daß der Schritt e) zwischen etwa 50° und 250 °C über einen Zeitraum, der von ungefähr 30 min bis ungefähr 1 Stunde läuft, durchgeführt wird, um aktive Ni$^+$-Stellen zu erhalten, deren Koordinationsphäre ein oder zwei Triethylphosphin-Liganden aufweist.

10. Verfahren nach einem der Ansprüche 1 bis 6, dadurch gekennzeichnet, daß der Schritt e) zwischen etwa 300 und 400 °C über einen Zeitraum, der von ungefähr 30 min bis ungefähr 1 Stunde läuft, durchgeführt wird, um aktive Ni$^+$-Stellen zu erhalten, deren Koordinationsphäre zwei Trimethylphosphin-Ligangen aufweist.

11. Verfahren nach einem der Ansprüche 1 bis 6, dadurch gekennzeichnet, daß der Schritt e) bei Raumtemperatur über einen Zeitraum von ungefähr einer Stunde durchgeführt wird, um aktive Ni$^+$-Stellen zu erhalten, deren Koordinationsphäre einen Tricyclohexylphosphin-Liganden aufweist.

FIG. 1

FIG_2

EP 0 209 436 B1

FIG. 3

FIG. 4

FIG. 5

0 : Ni$^+$

1 : Ni$^+$(PEt$_3$)

2 : Ni$^+$(PEt$_3$)$_2$

3 : Ni$^+$(PMe$_3$)$_2$

n-hexènes

méthylpentènes

diméthylbutènes

EP 0 209 436 B1